# EUROPEAN PATENT SPECIFICATION

(11) **EP 0 437 487 B1**
(45) Date of publication and mention of the grant of the patent: **09.03.1994**
(21) Application number: 89911284.1
(22) Date of filing: 04.10.1989
(51) Int. Cl.: A61K 47/48, A61K 37/32, C07K 99/10, C07K 7/24

(54) **TARGETTING AGENTS**
ZIELMITTEL
AGENTS CIBLANTS

(30) Priority: 04.10.1988 GB 8823208; 17.01.1989 GB 8900988; 23.06.1989 GB 8914526
(43) Date of publication of application: 24.07.1991
(73) Proprietor: CANCER RESEARCH CAMPAIGN TECHNOLOGY LIMITED, London SW1Y 5AR (GB)
(72) Inventor: PAGE-THOMAS, David, Peter, Cambridge CR1 4RU (GB); BARD, David, Roy, Wort's Causeway Cambridge CR1 4RU (GB); KNIGHT, Clive, Graham, Cambridge CR1 4RU (GB)
(74) Representative: Molac, Béatrice
(86) International application number: GB8901180
(87) International publication number: WO9003798

(56) References cited:
- EP-A- 0 094 844
- EP-A- 0 268 707
- Biochemical Society Transactions, volume 14, 1986, (London, GB), D.R. Bard et al.: "Targetting of a radionuclide to Cloudman melanoma cells in vitro and in vivo", pages 614-615, see the whole article cited in the application
- Proc. Natl. Acad. Sci, USA, volume 83, November 1986, (Washington, D.C., US)
- J.R. Murphy et al.: "Genetic construction, expression, and melanoma-selective cytotoxicitiy of a diphteria toxin-related a-melanocyte-stimulating hormone fusion protein", pages 8258-8262, see page 8258, paragraph 3; page 8261, paragraph 6 - page 8262, paragraph 2, cited in the application

## Description

The present invention relates to targetted agents directed against malignant melanoma cells and to their production and use.

Malignant melanoma occurs at an annual rate of between 1 and 6 cases per 100,000 population worldwide. It is particularly prevalent in the caucasian populations of Australasia, the U.S.A., Switzerland and Scandinavia and has shown a marked increase in incidence over the last two decades. Prognosis is poor in comparison with other dermal carcinomas due to the tendency of melanomas to early metastasis and their resistance to gamma irradiation and systemic cytotoxic chemotherapy. The success of therapy would be markedly improved by an agent which could target diagnostic or therapeutic agents to primary melanomas and their metastases.

Much work in this area has concentrated on the development of monoclonal antibodies to specific cell surface markers. Such an approach suffers from the difficulty of finding a cell surface marker unique to melanomas, but shared by all variants of the tumour and the recognition and elimination of the antibody molecule by the immune system of the patient.

An alternative approach utilises the observation that both malignant and normal melanocytes possess receptors for the peptide hormone alpha-melanocyte stimulating hormone (MSH). MSH stimulates the production of melanin by melanocytes in a wide range of chordate species. In mammals, it does so by combining with cell surface receptors, thus initiating a sequence of events which ultimately results in the stimulation of the synthesis of tyrosinase, the enzyme which converts tyrosine to L-DOPA, the immediate precursor of melanin.

MSH may be modified at the N-terminus with little or no loss in hormonal activity and MSH linked to diptheria toxin in this manner has been shown selectively to kill melanoma cells in vitro. (Murphy, J. R. et al., Proc. Natl. Acad. Sci. USA, 83, 8258-8262 (1986)). Similarly, MSH substituted with the chelator, diethylenetriamine pentaacetic acid (DTPA) at the N-terminus (MSH-DTPA), becomes rapidly associated with melanoma cells both in vitro and in vivo (Bard, D.R. et al., Biochem. Soc. Trans. London 14, 614-615 (1986)).

It has now surprisingly been discovered that new targetted agents which comprise two or more N-terminus-linked MSH-type moieties have improved binding to melanocytes and/or more rapid clearance from animals to which the agents have been administered.

Accordingly the present invention provides a product of formula (I)

R-(M)ₙ (I)

wherein n is greater than 1, preferably 2 or more, each M is a N-terminus-linked polypeptide selected from alpha-melanocyte stimulating hormone (MSH) and derivatives and analogues thereof and R is a targetted moiety optionally bonded to the groups M by a linking group.

The group R may be a directly bonded targetted moiety having at least two positions to which the N-termini of polypeptides may be attached without destroying the utility of the targetted moiety. Alternatively, the group R may be a bonded via a linking group which is itself bonded to the groups M. Targetted moieties with only one attachment site can be linked to bis-MSH by joining the two peptide chains through a linkage reagent that retains a reactive functional group. An example of such a reagent is the disuccinimidyl ester of succinyl-lysyl(Fmoc)-beta-alanine which contains a protected amino group.

Suitable targetted moieties include cytotoxic agents such as dacarbazine (5-[3,3-dimethyl-1-triazenyl]-1H-imidazole-4-carboxamide), actinomycin D, methotrexate and methyl CCNU (N-(2-chloroethyl)-N'-(trans-4-methylcyclohexyl)N-nitrosourea) and their precursors and cytokine agents such as tumor necrosis factor (TNF) and gamma interferon.

Other targetted moeities are detectable labels, such as radiolabels, for localisation and imaging of tumours. Particularly suitable radionuclides for use as cytotoxic agents include short half-life beta emitters such as erbium 169, yttrium 90, rhenium 186 and lutetium 177 and for use as imaging agents include indium 111, indium 113m and gallium 67.

In general, small targetted moieties such as radionuclides are unlikely to be suitable for direct bonding to the groups M and will, therefore, be bonded via a linking group. Suitable linking groups for binding many types of targetted moiety to peptides will be known to those skilled in the art. For linking radionuclides it is particularly preferred to use chelating agents such as diethylenetriamine pentaacetic acid (DTPA); ethylenediamine tetraacetic acid (EDTA); ethylene glycol-O,O'-bis(2-aminoethyl)-N,N,N',N'-tetraacetic acid (EGTA); triethylenetetramine hexaacetic acid (TTHA), and N,N'-bis(hydroxybenzyl)ethylenediamine-N,N'-diacetic acid (HBED).

The groups M may be the same or different but it is presently preferred that they are all the same. Each group M is an N-terminus-linked MSH peptide or an analogue or derivative thereof. Investigations by others have led to the identification of various analogues and derivatives of MSH which have improved binding to MSH receptors and it is particularly preferred to use N-terminus-linked MSH or a "super potent" synthetic melanotropin such as [Nle⁴, D-Phe⁷]-alpha-MSH described by Sawyer T.K. et al., Proc Natl Acad Sci USA 77, 5754-5788 (1980) and those described by F. Al-Obeidi et al., J. Med. Chem., 32, 174-179 (1989), particularly the super-potent MSH₄₋₁₀ analogues [Nle⁴, D-Phe⁷, Lys¹⁰, Gly¹¹] α-MSH₁₋₁₃-NH₂; [Nle⁴, Asp⁵, D-Phe⁷, Lys¹⁰, Gly¹¹] α-MSH₁₋₁₃-NH₂; [Nle⁴, D-Phe⁷, Lys¹⁰, Gly¹¹] α-MSH₄₋₁₃-NH₂; [Nle⁴, Asp⁵, D-Phe⁷, Lys¹⁰, Gly¹¹] α-MSH₄₋₁₃-NH₂; [Nle⁴, Asp⁵, D-Phe⁷] α-MSH₄₋₁₀-NH₂; [Nle⁴, D-Phe⁷, Lys¹⁰] α-MSH₄₋₁₀-NH₂; [Nle⁴, D-Phe⁷, Orn¹⁰] α-MSH₄₋₁₀-NH₂; [Nle⁴, Asp⁵, D-Phe⁷, Orn¹⁰] α-MHS₄₋₁₀-NH₂; [Nle⁴, D-Phe⁷, Dab¹⁰] α-MSH₄₋₁₀-NH₂; [Nle⁴, Asp⁵, D-Phe⁷, Dpr¹⁰] α-MHS₄₋₁₀-NH₂; [Nle⁴, Asp⁵ D-Phe⁷, Lys¹°] α-MSH₄₋₁₀ -NH₂ and [Nle⁴, Asp⁵ D-Phe⁷, Dab¹⁰, Lys¹⁰] α-MSH₄₋₁₀ -NH₂ and the cyclic α-melanotropins described in Al-Obeidi et al., J.Am.Chem.Soc., III, 3413-3417 (1989).

The products of the present invention may be mixtures or pure compounds. When they are mixtures, individual components of the mixture may have different values of n so that in the product as a whole, n has an average value which may be integral or fractional. Preferably the products of the invention are substantially pure compounds, in which case n will be an integer of 2 or more.

In another aspect the invention provides a product of formula (II)

L-(M)ₙ (II)

wherein M and n are as defined in relation to formula (I) and L is a linking group, preferably a chelating agent such as DTPA.

Particularly preferred compounds of formula (II) are 2 MSH-DTPA of formula (IIa) 
Ser-Tyr-Ser-Met-Glu-His-Phe-Arg-Trp-Gly-Lys-Pro-Val-NH2
DTPA
Ser-Tyr-Ser-Met-Glu-His-Phe-Arg-Trp-Gly-Lys-Pro-Val-NH2
(IIa)
and the analogues of formulae (IIb) to (IIm). 
Nle-Asp-His-D-Phe-Arg-Trp-Lys-NH2
DTPA (IIb)
Nle-Asp-His-D-Phe-Arg-Trp-Lys-NH2

Nle-Asp-His-D-Phe-Arg-Trp-Dab-NH2
DTPA (IIc)
Mle-Asp-His-D-Phe-Arg-Trp-Dab-NH2

Ser-Tyr-Ser-Nle-Glu-His-D-Phe-Arg-Trp-Lys-Gly-Pro-Val-NH2
DTPA (IId)
Ser-Tyr-Ser-Nle-Glu-His-D-Phe-Arg-Trp-Lys-Gly-Pro-Val-NH2

Ser-Tyr-Ser-Nle-Asp-His-D-Phe-Arg-Trp-Lys-Gly-Pro-Val-NH2
DTPA (IIe)
Ser-Tyr-Ser-Nle-Asp-His-D-Phe-Arg-Trp-Lys-Gly-Pro-Val-NH2

Nle-Glu-His-D-Phe-Arg-Trp-Lys-Gly-Pro-Val-NH2
DTPA (IIf)
Nle-Glu-His-D-Phe-Arg-Trp-Lys-Gly-Pro-Val-NH2
 
Nle-Asp-His-D-Phe-Arg-Trp-Lys-Gly-Pro-Val-NH2
DTPA (IIg)
Nle-Asp-His-D-Phe-Arg-Trp-Lys-Gly-Pro-Val-NH2

Nle-Asp-His-D-Phe-Arg-Trp-Gly-NH2
DTPA (IIh)
Nle-Asp-His-D-Phe-Arg-Trp-Gly-NH2

Nle-Glu-His-D-Phe-Arg-Trp-Lys-NH2
DTPA (IIi)
Nle-Glu-His-D-Phe-Arg-Trp-Lys-NH2

Nle-Glu-His-D-Phe-Arg-Trp-Orn-NH2
DTPA (IIj)
Nle-Glu-His-D-Phe-Arg-Trp-Orn-NH2

Nle-Asp-His-D-Phe-Arg-Trp-Orn-NH2
DTPA (IIk)
Nle-Asp-His-D-Phe-Arg-Trp-Orn-NH2
 
Nle-Glu-His-D-Phe-Arg-Trp-Dab-NH2
DTPA (IIl)
Nle-Glu-His-D-Phe-Arg-Trp-Dab-NH2
Nle-Asp-His-D-Phe-Arg-Trp-Dpr-NH2
DTPA (IIm)
Nle-Asp-His-D-Phe-Arg-Trp-Dpr-NH2
Products of the present invention may be made by conventional techniques. In order to link two or more peptides to a single targetted moiety or linking group, it will often be convenient to have the peptides bound to a solid substrate for instance the resin beads conventionally used in peptide synthesis.

The invention therefore further provides a process for producing a product of formula (I) comprising reacting a group R or a reactive derivative thereof with groups M or reactive derivatives thereof. Where the group R is a targetted moiety bonded via a linking group the process will generally involve the steps of (a) reacting a group L or a reactive derivative thereof with groups M or reactive derivatives thereof to form a product of formula (II) as hereinbefore defined and (b) reacting the product of formula (II), or a reactive derivative thereof with a compound R' or a reactive derivative thereof containing the targetted moiety R.

The reaction conditions such as temperature, duration and selection of solvents and the nature of the reactive derivatives will be determined by the nature of the groups R, L and R' and it is within the ability of the skilled person to select these as appropriate.

Preferably the process further involves separation and purification of the product. For products where the targetted moiety R is bonded via a linking group L which is a chelating agent such as DTPA, the production process preferably involves: synthesis of the peptide chains on a resin substrate by sequential addition of protected amino acid residues, deprotection as necessary and then reacting the peptide chains with the linking reagent before removing the peptide chains from the resin substrate and finally purifying the agent so produced, for instance by reverse phase chromatography.

Products according to the present invention are useful in the diagnosis and surgical or therapeutic treatment of melanoma, for instance in imaging tumours and delivery of drugs to the tumour sites. The present invention therefore further provides a product of formula (I) or (II) for use in a method of treatment of the human or animal body by surgery or therapy or in a method of diagnosis practised on the human or animal body. Also provided are diagnostic methods using the products of formula (I) or (II) in testing samples of tissue removed from the human or animal body. The invention further provides the use of a compound of formula (I) as a pharmaceutical for the treatment or diagnosis of melanomas.

The products of formula (I) or (II) may be used directly or they may be administered in the form of a pharmaceutical composition comprising the product of formula (I) or (II) and a pharmaceutically acceptable diluent or carrier.

Such diluents and carriers include tabletting agents, wetting agents, binders and fillers, preservatives such as antioxidants and antimicrobial agents, buffers and salts as well as conventional injection media, particularly water for injection. Such pharmaceutical compositions, and processes for formulating them, form further aspects of the present invention as does the use of a product of formula (I) or (II) in the preparation of a medicament for use in the diagnosis or surgical or therapeutic treatment of melanoma.

The products of formula (I) or (II), or compositions thereof, are administered by any conventional route including, enteral and parenteral routes especially by injection by the intravenous, intramuscular, subcutaneous or peritoneal routes.

Dosages and dosage regimes will depend on whether the agent is being administered for localisation or imaging purposes or as a therapeutic agent and on the age, health, weight and sex of the recipient. Typical dosages are likely in the region of from 0.001mg/kg to 0.05 mg/kg per day, preferably from 0.003 mg/kg to 0.02 mg/kg per day. For adult humans a normal daily dose is envisaged of from 0.05 mg to 5 mg, preferably from 0.01 mg to 1 mg, for instance 0.5 mg. This dose may be administered as a single dose or as divided doses at intervals. Dosages would be repeated as necessary.

The invention will now be illustrated by the following Figures of the accompanying drawings and by the following non-limiting Examples.

In the accompanying drawings:
Fig. 1 shows a comparison of MSH, Des-acetyl MSH, MSH-DTPA and 2MSH-DTPA in the tyrosinase assay.
Fig. 2 shows ¹¹¹In labelled 2 MSH-DTPA concentrations, in various tissues, following injection into mice at intervals.
Fig. 3 shows a comparison of tumour/blood concentration ratios for 2 MSH-DPTA, MSH-DTPA and indium citrate 42 h after injection into mice.
Fig. 4 illustrates the whole-body clearance rates for 2MSH-DTPA, MSH-DTPA and indium citrate following injection into rats.
Fig. 5 shows the results of (a) Anterior and (b) Posterior whole body I.G.E. scans at 4hrs and 5 mins.
Fig. 6 shows a spot I.G.E. scan of the neck and head (Anterior) at 4 hrs and 5 mins.
Fig. 7 shows the results of (a) Anterior and (b) Posterior whole body I.G.E. scans at 48 hrs.
Fig. 8 is a graph of whole body retention of 2 MSH-DTPA calculated from the probe measurements, the whole body I.G.E. scans and the radioactivity detected in the urine.
Fig. 9 is a graph of blood clearance of 2 MSH-DTPA
Fig. 10 is a graph of radioactivity associated with tumour, kidneys, liver, lung and spleen expressed as proportions of the whole body radioactivity at zero time. The figures are not corrected for the mass of each organ.
Fig. 11 is a graph of radioactivity associated with tumour, kidneys, liver, lung and spleen expressed as proportions of the whole body radioactivity remaining at each time point. The figures are not corrected for the mass of each organ.
Fig. 12 is a graph of radioactivity in tumour, kidneys and liver calculated as absolute counts per unit volume of tissue. The volumes were calculated as (area on scan)^{3/2} and the results are expressed relative to each other on an arbitrary scale.

### EXAMPLE 1

### A) SYNTHESIS OF 2MSH-DTPA

The peptide was synthesised step-wise from the C-terminus by the Fmoc-polyamide method using standard procedures on a Cambridge Research Biochemicals Pepsynthesiser (cf. Dryland, A. & Sheppard, R.C. Tetrahedron 44, 859-876 (1988)). Pepsyn K resin (2g, 0.1 mmoles of norleucine/g) was used and the linkage agent was Fmoc-amino-(2,4-dimethoxy-4'-carboxymethoxy)diphenylmethane. The Fmoc group of the linkage agent was removed and the resulting amino-resin was treated with Fmoc-valine pentafluorophenyl ester and 1-hydroxy-benzotriazole (0.8 mmoles of each). After 30 min, coupling to the amine was complete and the resin was washed prior to removal of the Fmoc group and the coupling of the next residue. This procedure was repeated at each step using Fmoc amino acid pentafluorophenylesters, except in the case of serine which was coupled as the oxobenzotriazine ester. Side chains were protected as tert-butyl derivatives, except for arginine where the 4-methoxy-2,3,6-trimethylbenzenesulphonyl group was used.

When the assembly of the peptide was complete, the Fmoc group was removed from the N-terminal residue and the peptide-resin was reacted with DTPA bis-anhydride (108 mg, 0.3 mmoles) in the presence of diisopropylethylamine (0.025 mL, 0.15 mmoles). Reaction was complete after 1 hr and the resin was washed with dimethylformamide (DMF), water, DMF, dichloromethane and ether. After drying, a portion of the resin (0.7 g) was treated overnight with a mixture of trifluoroacetic acid (TFA), ethane-dithiol, phenol and anisole (97:1:1:1, by vol.).

Evaporation of the TFA solution followed by trituration with ether yielded a white solid (110mg). This was purified by reverse phase chromatography on a column (15 mm x 500 mm) of Vydac C18 (15 - 20 um) in a low pressure system. A linear gradient was applied from 0.1% TFA in water to 35% (v/v) acetonitrile in 0.1% TFA over 1.5 L. Fractions containing 2MSH-DTPA were identified by high pressure liquid chromatography and combined and lyophilised to yield 11 mg. The identity of the product was confirmed by fast atom bombardment mass spectrometry which gave a relative molecular mass of 3602 ± 2.

### B) PREPARATION OF ¹¹¹ INDIUM LABELLED 2MSH-DTPA

Peptide (0.5 mg) was dissolved in water (0.5ml) and citrate buffer (0.1M, pH 5.6, 1.0ml) was added. This solution was labelled with ¹¹¹InCl₃ (1.0 MCi in 0.1 ml 0.04M hydrochloric acid (Amersham)). Binding to the peptide was confirmed by thin-layer chromatography on silica gel using a double ascent system. The first solvent was 10% aqueous ammonium acetate/methanol (1:1, v/v) and the second, methanol/water (8.5:1.5, v/v). In this system unbound indium forms a colloidal hydroxide which has an Rf of 0.5, whilst labelled peptide moves at the solvent front. 100% of the radioactivity was located at the solvent front when the solution was analysed immediately after the addition of indium, and after standing for 5h at 18°C.

### C) TYROSINASE ACTIVITY IN VITRO

Cloudman S91 cells were grown in monolayers to a density of approximately 25,000 cells cm⁻² in 25cm² culture flasks using 4 ml Ham's F10 medium supplemented with 10% heat inactivated foetal calf serum. The medium was replaced and 10⁻⁵ and 10⁻⁹ molar concentrations of MSH, des-acetyl MSH, MSH-DTPA or 2MSH-DTPA added. After 24 h, medium and melantropins were renewed and 1uCi, 3,5-³H-tyrosine included. The final specific activity of 3,5-³H-tyrosine incubation medium was 0.1 Ci/mmol.

After a further 24h, 0.2ml of the medium from each incubation were transferred to centrifuge tubes containing 1ml activated charcoal suspension (100 mg/ml in 0.1M citric acid). The tubes were vortexed and centifuged at 700 x g for 5 min. Aliquots (0.5 ml) of each supernatant were transferred to scintillation vials and counted on a Packard Tri-Carb 300D scintillation counter. Aliquots (0.1ml) of the unextracted medium were also counted. Finally, the remaining medium was removed and the cell monolayer suspended in 1ml isotonic trypsin/EDTA. The dispersed cells were counted in a Coulter counter.

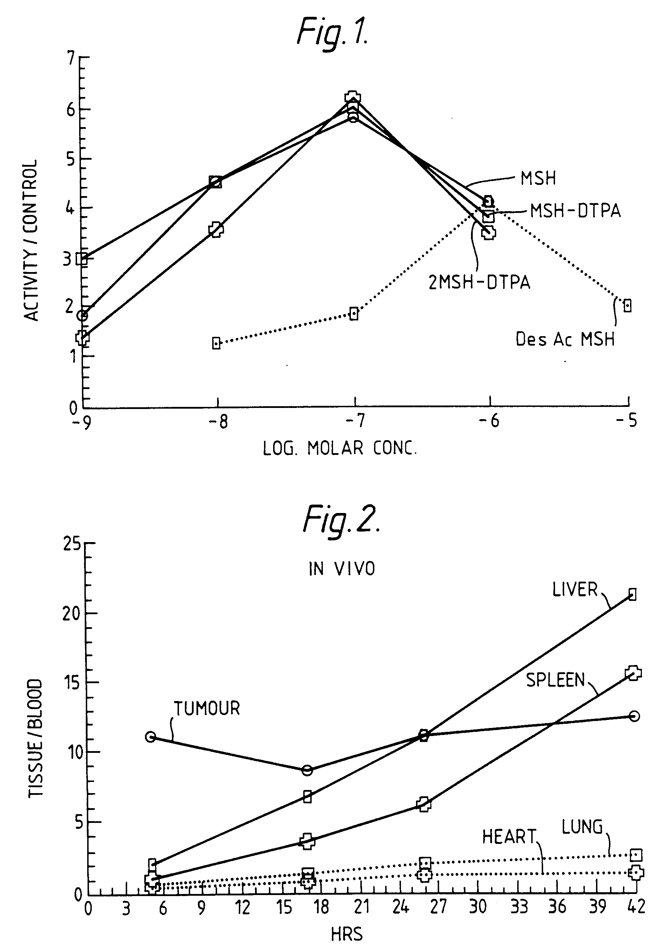

The amount of water generated from the tyrosine per 10⁵ cells was calculated and the results divided by the value obtained from the melanotropin-free control. Results are shown in Fig. 1.

### D) COMPARISON TESTING IN VIVO

2MSH-DPTA or MSH-DTPA was labelled with ¹¹¹In by the general method described in (B) but using 2.5 nmol DTPA-peptide (0.277 pg 2MSH-DTPA, 0.153 pg MSH-DTPA) in 0.05 ml water, 0.2 ml citrate buffer and 5uCi ¹¹¹In. The solution was adjusted to 2.5ml after the addition of indium with physiological saline. 0.1ml of this solution was injected i.p. into 20 DBA2 mice which had been innoculated with Cloudman S91 cells i.p. 14 days previously. In some experiments, mice were also injected with a solution prepared as above, but with the omission of DTPA-peptide.

Animals were killed in groups of 5 at 5, 17, 26 and 42h after injection of the labelled peptide. Blood (0.1-0.3 g), brain (whole), eyes, liver (approx. 1g) spleen (whole), heart (whole), lungs (whole), skeletal muscle (approx. 0.1g) and tumour (0.1-0.5g) were removed. The individual tissues were weighed and counted on a Packard Multi-Prias 4 gamma scintillation counter using a window of 50-500 KeV. Results were expressed as CPM/g wet weight and divided by the blood value at each time point.

For the whole body retention experiments, 0.5 ml each of the three radioactive preparations already described were injected i.p. into normal rats. Radioactivity was measured on the sedated animals by an external gamma scintillation probe positioned at the anterior of the animal and facing along the axis of the animals so that the whole body was within the cone of acceptance of the probe. Measurements were taken at 0, 4, 21, 46 and 72 hours after injection and the results expressed as a proportion of the maximum counts detected.

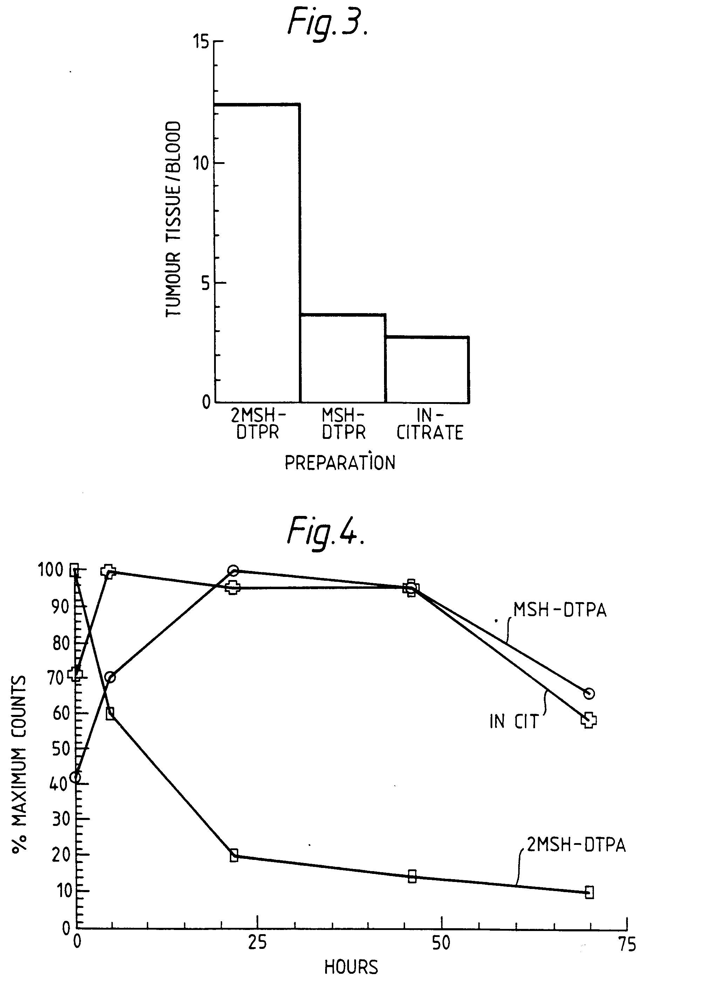

Results are shown in Figs. 2 to 4.

### EXAMPLE 2

Synthesis of bis(Nle⁴, Asp⁵, D-Phe⁷, Lys¹⁰) α-MSH₄₋₁₀ - DTPA.

The peptide was synthesised step-wise from the C-terminus by the Fmoc-polyamide method using standard procedures on a Cambridge Research Biochemicals Pepsynthesiser (cf., Dryland, A. & Sheppard, R.C. Tetrahedron 44, 859-876 (1988)). Pepsyn KB resin (1 g, 0.2 mmoles of norleucine/g) was used and the linkage agent was Fmoc-amino-(2,4-dimethoxy-4'-carboxymethoxy)diphenylmethane. The Fmoc group of the linkage reagent was removed and the resulting amino-resin was treated with Fmoc-lysine(Boc) pentafluorophenyl ester and 1-hydroxybenzotriazole (0.6 mmoles of each). After 1 hr, coupling to the amine was complete and the resin was washed prior to removal of the Fmoc group. The next residue, tryptophan, was also added as the pentafluorophenyl ester. The third and fourth residues, arginine and D-phenylalanine, were coupled as free acids in the presence of benzotriazol-1-yloxy-tris(dimethylamino)phosphonium hexafluorophosphate (BOP) and di-isopropylethylamine (0.6 mmoles of each reagent). The remaining residues were added as the pentafluorophenyl esters. Side chains were protected as tert-butyl derivatives, except for arginine, for which the 2,2,5,7,8-pentamethylchroman-6-sulphonyl group was used.

When the assembly of the peptide was complete, the Fmoc group was removed from the N-terminal residue and the peptide resin was reacted with DTPA bis-anhydride (55 mg, 0.15 mmoles) in the presence of 1-hydroxybenzotriazole and di-isopropylethylamine (0.15 mmoles of each). After standing overnight the resin was washed with dimethylformamide (DMF) and the reaction was repeated with fresh reagents. Reaction was complete 24 hrs later and the resin was washed with DMF, water, DMF, acetic acid and ether. After drying, the resin (1.21 g) was gently stirred with a mixture of trifluoroacetic acid (58 ml), anisole (1 ml), ethanedithiol (0.5 ml) and indole (1 g). After 2 hrs, the resin was filtered, washed with TFA and the combined filtrates rotary evaporated. Ether (100 ml) was added to the residue to precipitate the crude peptide (173 mg). The product was purified by reverse phase chromatography on Vydac C18. The compound was labelled with In¹¹¹ as described in Example 1.

### EXAMPLE 3

Synthesis of bis(Nle⁴, Asp⁵, D-Phe⁷, Dab¹⁰) MSH₄₋₁₀ - DTPA.

This was made and purified exactly as described above, except that the first amino acid was L-diaminobutyric acid (Dab). The compound was labelled with In¹¹¹ as described in Example 1.

### EVIDENCE OF TARGETTING TO A HUMAN MELAMONA

The patient was a 57 year old man with a four year history of melanoma. A primary tumour in the left eyelid (surgically removed) had metastasised, forming large nodes in the left side of the neck. The patient was receiving chemotherapy.

2MSH-DTPA, 0.5mg, labelled with 35 MBq ¹¹¹In was given by slow intravenous infusion over a few minutes. Whole body radioactivity was measured immediately after infusion by means of a probe. Anterior and posterior whole body and tumour area spot I.G.E. scans (with computer data acquisition) and whole body probe measurements were carried out at 4 h, 24 h and 48 h after infusion. Heparinised blood samples, 10ml, were taken at 10 min, 2 h, 4 h, 24 h, and 48 h and urine was collected from 0 - 4 h, 4 - 24 h and 24 - 48 h.

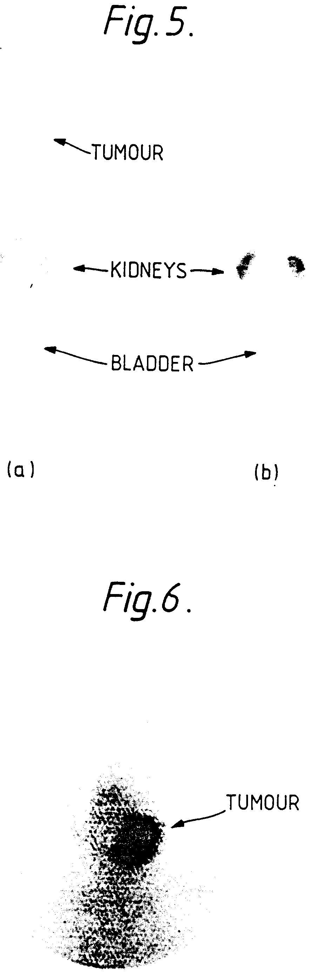

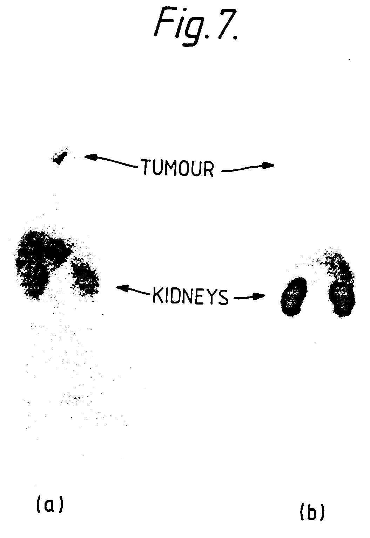

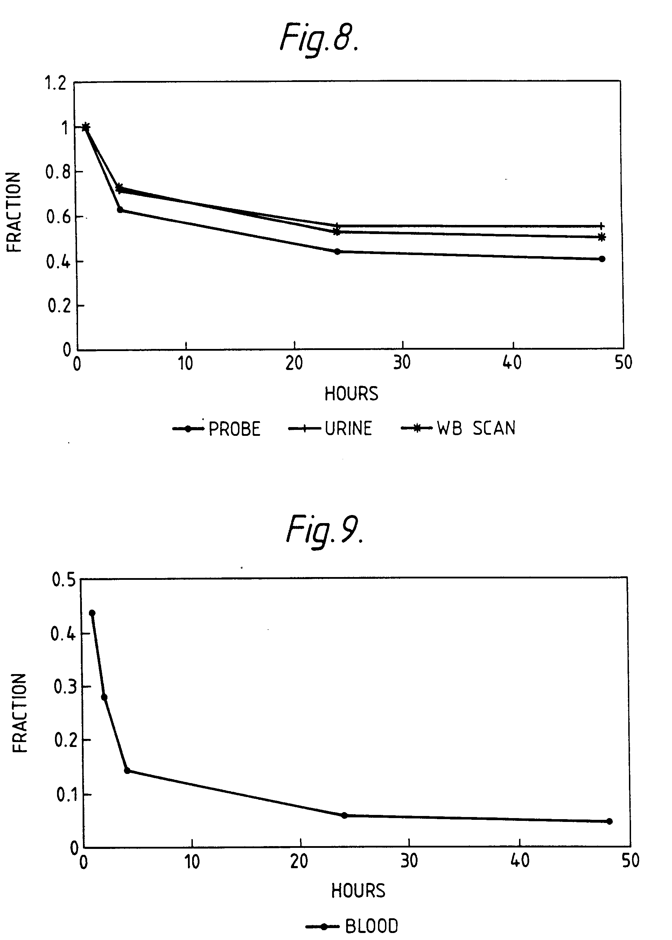

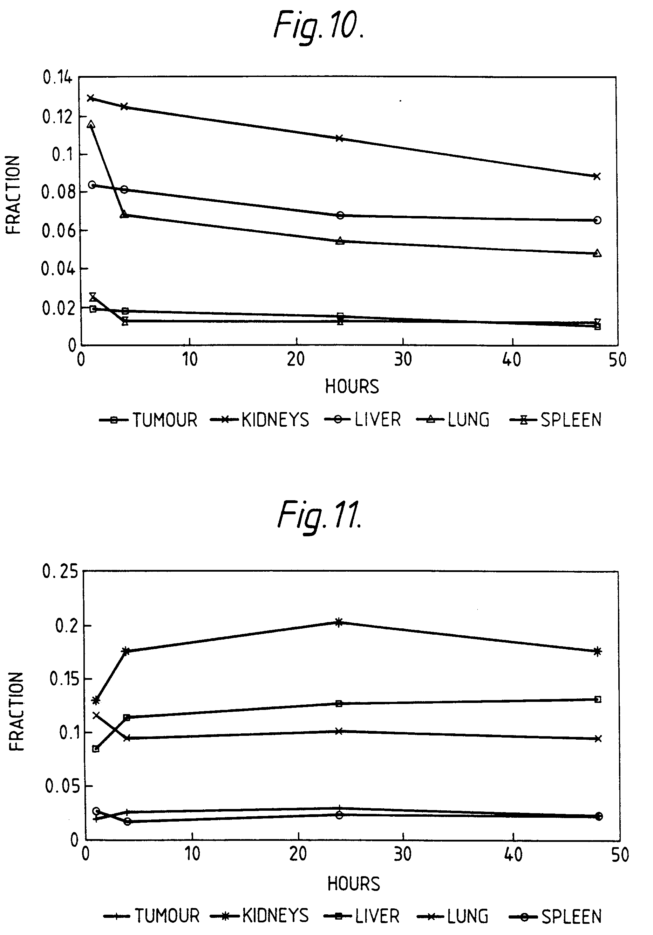

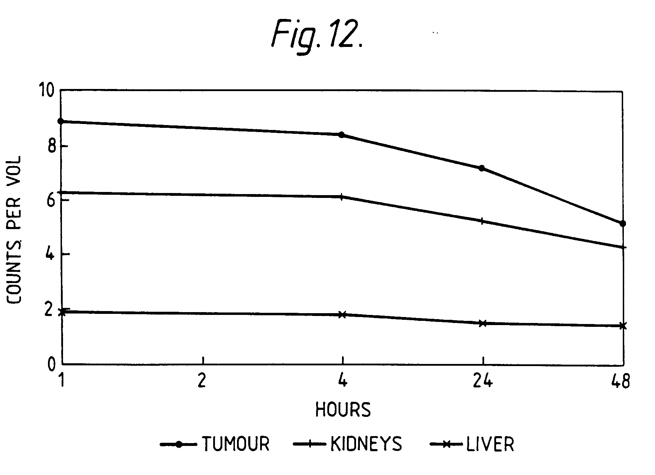

Results are shown in Figs. 5 to 12.

## Claims

1. A compound of formula (I)
R-(M)ₙ (I)
wherein n is greater than 1, each M is a N-terminus-linked polypeptide selected from alpha-melanocyte stimulating hormone (MSH) and derivatives and analogues thereof having binding affinity to MSH receptors and R is a targetted moiety selected from a cytostatic agent, a precursor therefor, a cytokine agent and a radionuclide,
R being bonded to the groups M by a linking group capable of chelating a radionuclide when R is a radionuclide.

2. A compound according to claim 1 wherein R is attached to the groups M through a linking group capable of joining R with the M terminal amino groups when R is other than a radionuclide.

3. A compound according to claim 1 or 2 wherein n is 2.

4. A compound according to any one of claims 1 to 3 wherein the groups M are the same and each is N-terminus-linked α-melanocyte stimulating hormone (MSH) or a super-potent synthetic melanotropin selected from
[Nle⁴, D-Phe⁷]-α-MSH;
the super-potent MSH₄₋₁₀ analogues of MSH and cyclic α-melanotropins.

5. A compound according to any one of claims 1, 3 and 4, wherein R is a radionuclide bonded to the groups M through a linking group capable of chelating the radionuclide.

6. A compound according to claim 5 wherein R is a radionuclide for localisation and imaging of tumours or a β-emitting radionuclide.

7. A compound according to claim 5 or 6 wherein R is bonded to the groups M through diethylenetriamine pentaacetic acid.

8. A compound of formula (II)
L-(M)ₙ (II)
wherein M and n are as defined in claim 1 and L is a linking group capable of chelating a radionuclide.

9. A process for producing a compound of formula (I) according to any one of claims 1 to 7 which comprises reacting a group R or a reactive derivative thereof with groups M or reactive derivatives thereof.

10. A pharmaceutical formulation comprising a compound of formula (I) or II according to any one of claims 1 to 8 and a pharmaceutically acceptable diluent or carrier thereof.

11. A compound according to any one of claims 1 to 8 for use in the preparation of a medicament for use in the diagnosis or surgical or therapeutic treatment of melanoma.

## Patentansprüche

1. Eine Verbindung der Formel I
R-(M)ₙ (I)
worin n grösser als 1 ist, jedes M ein N-terminus-gebundenes Polypeptid ausgewählt aus dem α-Melanocyten-stimulierenden Hormon (MSH) und Derivaten und Analoga davon, welche eine Bindungsaffinität zu MSH Rezeptoren haben, und R ein zielgerichteter Teil ausgewählt aus einem cytostatischen Mittel, einem Vorgänger davon, einem Cytokin-Mittel und einem Radionuklid ist,
R und die Gruppen M durch eine Verknüpfungsgruppe gebunden sind, welche fähig ist, ein Chelat mit einem Radionuklid zu bilden, wenn R ein Radionuclid ist.

2. Eine Verbindung gemäss Anspruch 1, worin R an die Gruppen M durch eine Verknüpfungsgruppe gebunden ist, welche fähig ist R mit den Endaminogruppen von M zu verbinden, wenn R anders als ein Radionuklid ist.

3. Eine Verbindung gemäss Anspruch 1 oder 2, worin n für 2 steht.

4. Eine Verbindung gemäss irgendeinem der Ansprüche 1 bis 3, worin die Gruppen M gleich sind und jeweils ein N-terminus-gebundenes α-Melanocytenstimulierendes Hormon (MSH) oder ein N-terminus-gebundenes superwirksames synthetisches Melanotropin ausgewählt aus
[Nle⁴, D-Phe⁷]-α-MSH;
superwirksamen MSH₄₋₁₀ Analoga von MSH und cyklischen α-Melanotropinen, ist.

5. Eine Verbindung gemäss irgendeinem der Ansprüche 1, 3 und 4, worin R ein Radionuklid, das an die Gruppen M durch eine Verknüpfungsgruppe gebunden ist, welche fähig ist, ein Chelat mit dem Radionuklid zu bilden.

6. Eine Verbindung gemäss Anspruch 5, worin R ein Radionuklid zur Lokalisierung und Abbildung von Tumoren oder ein β-emitierendes Radionuklid ist.

7. Eine Verbindung gemäss Anspruch 5 oder 6, worin R an die Gruppen M durch Diethylentriamin Pentaessigsäure gebunden ist.

8. Eine Verbindung der Formel II
L-(M)ₙ (II)
worin M und n wie in Anspruch 1 definiert sind und L für eine Verknüpfungsgruppe steht, die fähig ist, ein Chelat mit einem Radionuklid zu bilden.

9. Verfahren zur Herstellung einer Verbindung der Formel I gemäss irgendeinem der Ansprüche 1 bis 7, das die Umsetzung einer Gruppe R oder eines reaktiven Derivates davon mit Gruppen M oder reaktiven Derivaten davon umfasst.

10. Pharmazeutische Zusammensetzung enthaltend eine Verbindung der Formel I oder II gemäss irgendeinem der Ansprüche 1 bis 8 zusammen mit einem pharmazeutisch annehmbaren Trägerstoff oder Verdünnungsmittel.

11. Eine Verbindung gemäss irgendeinem der Ansprüche 1 bis 8 zur Verwendung in der Herstellung eines Arzneitmittels zur Verwendung in der Diagnose oder chirurgischen oder therapeutischen Behandlung von Melanomen.

## Revendications

1. Un composé de formule I
R - (M)ₙ (I)
dans laquelle n est supérieur à 1, chaque M est un polypeptide lié à l'extrémité N choisi parmi l'hormone mélanostimulante α(MSH) et ses dérivés et analogues ayant une affinité de liaison aux récepteurs MSH et R est un reste ciblant choisi parmi les agents cytostatiques, leurs précurseurs, les cytokines et les radionucléides,
R étant lié aux groupes M par un groupe de jonction capable de former un chélate avec une radionucléide lorsque R signifie un radionucléide.

2. Un composé selon la revendication 1, dans lequel R est fixé aux groupes M par l'intermédiaire d'un groupe de jonction capable de réunir R aux groupes amino terminaux de M lorsque R est autre qu'un radionucléide.

3. Un composé selon la revendication 1 ou 2, dans lequel n signifie 2.

4. Un composé selon l'une quelconque des revendications 1 à 3, dans lequel les groupes M sont identiques et signifient chacun l'hormone mélanostimulante α(MSH) liée à l'extrémité N ou une mélanotropine synthétique super-active liée à l'extrémité N et choisie parmi
[Nle⁴, D-Phe⁷]-α-MSH
les analogues MSH₄-₁₀ superactifs de la MSH et les α-mélanotropines cycliques.

5. Un composé selon l'une quelconque des revendications 1, 3 et 4, dans lequel R est un radionucléide lié aux groupes M par l'intermédiaire d'un groupe de jonction capable de former un chélate avec le radionucléide.

6. Un composé selon la revendication 5, dans lequel R est un radionucléide pour la localisation et l'imagerie des tumeurs ou un radionucléide à émission β.

7. Un composé selon la revendication 5 ou 6, dans lequel R est lié aux groupes M par l'intermédiaire de l'acide diéthylène-triamine-penta-acétique.

8. Un composé de formule II
L-(M)ₙ (II)
dans laquelle M et n sont tels que définis à la revendication 1 et L est un groupe de jonction capable de former un chélate avec un radionucléide.

9. Un procédé de préparation d'un composé de formule I selon l'une quelconque des revendications 1 à 7, qui comprend la réaction d'un groupe R ou l'un de ses dérivés réactifs, avec des groupes M ou leurs dérivés réactifs.

10. Une composition pharmaceutique comprenant un composé de formule I ou II selon l'une quelconque des revendications 1 à 8 et un diluant ou véhicule pharmaceutiquement acceptable.

11. Un composé selon l'une quelconque des revendications 1 à 8 pour l'utilisation dans la préparation d'un médicament destiné à être utilisé dans le diagnostic ou le traitement chirurgical ou thérapeutique des mélanomes.
